# EUROPEAN PATENT SPECIFICATION

(11) **EP 3 498 237 B1**
(45) Date of publication and mention of the grant of the patent: **15.07.2020**
(21) Application number: 18160600.5
(22) Date of filing: 07.03.2018
(51) Int. Cl.: A61F 5/56, A61F 5/08

(54) **NASAL DEVICE TO INCREASE AIRFLOW**
NASENVORRICHTUNG ZUR ERHÖHUNG DER LUFTSTRÖMUNG
DISPOSITIF NASAL AFIN D'AUGMENTER LA CIRCULATION D'AIR

(30) Priority: 18.12.2017 US 201715845461
(43) Date of publication of application: 19.06.2019
(73) Proprietor: Pona, Adrian, Tecumseh, Ontario N8N 4N9 (CA)
(72) Inventor: Pona, Adrian, Tecumseh, Ontario N8N 4N9 (CA)
(74) Representative: Maiwald Patent- und Rechtsanwaltsgesellschaft mbH

(56) References cited:
- WO-A1-2005/018506
- AU-B4- 2004 100 927
- US-A- 5 601 594
- US-A1- 2004 261 791
- US-A1- 2007 191 876
- US-A1- 2013 338 700

## Description

### FIELD OF THE INVENTION

The present invention relates to medical devices for increasing ventilation through the nasal canal, obstruction relief, preoperative and postoperative surgical molds (support), allergy relief, reduction of snoring and mouth breathing and supporting nasal passage. Document US 2013/338700 A1 is regarded as the closest prior art.

### BACKGROUND OF THE INVENTION

Anatomically, the nose is split into two passages by the nasal septum. The nasal septum is made up of bone, cartilage and epithelium. Sometimes the nasal septum may deviate and bend to one passageway causing partial or complete obstruction. Obstruction leads to difficulty in breathing, forcing the patient to breathe through the mouth. Increased breathing through the mouth can cause an assortment of secondary ailments. Future complications include difficulty in swallowing (dysphagia), dental caries, mucous irritation and difficulty in speaking (dysphonia). Surgeons may fix this by performing septoplasty. Septoplasty is a surgical procedure intended to straighten the septum by reshaping the cartilage and bone. A complication of septoplasty includes deviation of the septum because the cartilage has a memory of how it was positioned previously.

Rhinoplasty is the procedure used to enhance the function and aesthetics of the nose. The procedure is done by separating the nasal skin and soft tissue from the cartilaginous framework of the nose. The surgeon reconstructs the nose, sutures the incisions and places a stent into the nose for proper healing. Complications of rhinoplasty include deviation, adhesions and/or collapse of the airway canal.

Snoring is defined as the bitter sound that occurs from anatomical structures vibrating when someone sleeps. The cause of snoring is due to the vibration of the relaxed soft palate and uvula. The vibrating object causes waves to propagate into a medium forming sound, which is enhanced by mouth breathing. Other causes include alcohol, sleeping in a supine position causing the dorsal part of the tongue to collapse and obstructive sleep apnea characterized by the soft tissue collapsing secondary to connective tissue deposition in the pharynx in people suffering from obesity or acromegaly.

When patients have difficulty breathing through their nostril, they are forced to ventilate with their mouth. Ventilation through the mouth may lead to snoring, throat soreness after sleeping and dry mouth.

A complication that may be caused by nasal obstruction is the lack of breathing through the nose while eating. The lack of breathing through the nose may lead to choking and death.

The nostril passageway is a canal that allows the travel of atmospheric air into the lungs for oxygen and carbon dioxide exchange between the alveoli and capillaries. The passageway enters the nasal cavity via the superior, middle and inferior nasal meatus. The nasal meatus are made by medial invaginations of bone called the turbinates or conchae. Four paranasal sinuses (maxillary, frontal, ethmoidal and sphenoidal) open and drain into the nasal cavity. The function of the nasal cavity and paranasal sinuses are to warm up the air, prevent irritation and act as an immunological defense against microorganisms.

Sinusitis is defined as the inflammation of the paranasal sinus. Sinusitis is classified into acute and chronic. Chronic sinusitis is defined as inflammation of the sinus for an extended period of time, from weeks to years. Causes of chronic sinusitis include polyps, deviated nasal septum and nasal passage stenosis (narrowing). Increasing the diameter of the nasal passage or surgically excising the polyp may prevent chronic sinusitis.

Increasing the rate of breathing through the nose may help prevent pharyngeal irritation during the fall and winter season. This may include outdoor hobbies, social activities and sports.

A study has been made regarding children's intelligence. The proposed study states that a child who sleeps while breathing through their nose will develop more intellectually in relation to a child who sleeps while breathing through their mouth.

Nasal dilator strips with an adhesive void (US005549103A) has the function of opening the anterior nasal aperture and increasing ventilation through the nostril. Side effects of nasal dilator strips include skin irritation from the adhesive, contact dermatitis, social embarrassment and temporary widening of the exterior nose.

Best, US Patent 5,601,594 provides a nasal stent for packing the nasal cavity following surgery, which includes a deformable cylinder with a breathing passageway therethrough, the cylinder having a diameter slightly larger than that of the nasal cavity of a user at an entranceway into the nasal cavities of the nose, wherein the cylinder has a smooth outer substantially non-absorbent surface that returns to its original shape following deformation. However, the Best nasal stent has a relatively thick radial material dimension that narrows the air passage. Additionally the Best nasal stent is larger than the nasal passage and must be compressed to be placed into the nose.

It is desirable to provide a device to increase air flow in a nasal passage without the disadvantages of prior nasal devices.

### SUMMARY OF THE INVENTION

To make manifest the above noted and other gainful desires, a revelation of the present invention is brought forth. In a preferred embodiment, the present invention endows a freedom of a device to increase airflow in a nasal passage comprising a tubular member being open at its extreme cylindrical ends. The tubular member having a flexible preform shape. The tubular member has a diameter smaller than a nasal passage accepting the tubular member. The tubular member is fabricated at least in part by an expandable polymeric material having a thickness equal to or less than 2mm and a length equal to or less than 40mm.

The nasal device of the present invention has a vast horizon of functions that are suitable for any person. The whole idea that separates previous nasal devices from this device is the incorporation of a flexible (and/or semi-rigid) and expandable walls. This device expands intra-nasally once placed in the proper position. The structure of the device can be solid, porous or mesh-like. The polymer strands can be crisscrossed so that they may expand to a preformed maximal shape, thereby pushing the walls outward of the nasal passage to open wider and allow easier flow of air. The nasal device of the present invention is easily removed when not needed.

The nasal device of the present invention is focused on opening the airflow of the interior nasal passage. By assessing the contour of the canal, the nasal device of the present invention can be manipulated to hold a patient's nasal airway. This allows the nasal device of the present invention to perfectly align inside the nasal passage. By taking this principle, the aim is to, from within, expand the physical pathway of the flow of air.

A patient's nasal passage can be scanned with a digital scanner and with a computer program, the nasal device of the present invention can then be custom designed for optimal shape of the nasal passage. Therefore, physicians and surgeons may make a custom nasal device that reflects the inside of the nose and reconstruct an optimal nasal canal after the surgery. In this case, the nasal device of the present invention would not be perforated as to avoid tissue growth through the perforations. This may help prevent nasal septal deviation after septoplasty, nasal collapse after rhinoplasty and act as an exterior hemostatic device. At the same time, the patient has the comfort to breathe naturally through the nose. Forget the uncomfortable; one size fits all, nasal packing. The sole purpose of this nasal device is to mold the interior nasal passage and prevent obstructive airways postoperatively.

The functions of the nasal device of the present invention are numerous. Firstly, this unique nasal device of the present invention allows constant passage of air through the nasal canal. The constant passage of air helps to warm the air, prevent dry mouth with future complications and increase immunologic response to foreign particles. It may be used for athletes to increase their oxygen demand and improve their fatigue, athleticism and skill. People who live in high altitudes will have a decrease in atmospheric oxygen levels. The dilation and increased ventilation can help with the intake of oxygen for metabolic purposes. Collapse or narrowing of the nasal passage affects the wellbeing of the person. A narrow nasal passage gives the person limitation that should not be present physiologically.

Secondly, the nasal device of the present invention helps to prevent obstructions that may be found postoperatively. Any surgery has consequences. After rhinoplasty, examples of complications include collapse of the lateral nasal cartilagedue to weakening after surgery and adhesion formation. This develops an obstruction in the nasal passage. The nasal device of the present invention may enter the nostril, expand the collapsed airway, relieve the obstruction and increase ventilation. It may also help prevent adhesion formation. Patients with a history of rhinoplasty and fall in the category of, nocturnal obligate nasal breathers, may have difficulty sleeping due to the collapse of the lateral nasal cartilage. This is an example of how the device may increase the wellbeing of the patient.

Thirdly, surgeons may incorporate the nasal device of the present invention in forming a precise picture of the nasal passage of the patient after the surgery. The nasal device can then be adjusted by the surgeon to the desired shape of the airway. This gives the surgeon complete control in preventing future complications postoperatively.

Lastly, people who breathe through their mouth, snore, suffer from sleep apnea or allergies can be aided by this device. This helps open up the nasal passage and increase ventilation, through the nose, therefore alleviate or diminish symptoms. It will offer the opportunity of a good night sleep. This will optimize the individuals and their families' quality of life.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:
Figure 1 is a perspective view of a preferred embodiment nasal device of the present invention;
Figure 2 is a perspective view of an alternate preferred embodiment nasal device of the present invention having crisscrossed wire reinforcement with a polymeric material coating;
Figure 3 is a perspective view of another alternate preferred embodiment nasal device of the present invention with crisscrossed strands of polymeric material;
Figure 4 is a front plan environmental view of a crescent-shaped alternate preferred embodiment nasal device of the present invention positioned in the nasal epithelium, cartilage and bone of a patient;
Figure 5 is a side elevational view of another alternate preferred embodiment nasal device of the present invention, on the floor of the nasal cavity;
Figure 6 is a perspective view of another alternate preferred embodiment nasal device of the present invention with a spiral reinforcement with a polymeric material with perforations;
Figure 7 is a perspective view of a tubular reinforcement for a nasal device of the present invention;
Figure 8 is a perspective view of a custom-made nasal device according to the present invention;
Figure 9 is a sectional view taken along lines 9-9 of Figure 8;
Figure 10 is a sectional view taken along lines 10-10 of Figure 8;
Figure 11 is a sectional view of the nasal device of the present invention shown in Figure 9 after expansion within a nasal cavity of a patient;
Figure 12 is a sectional view of an alternate preferred embodiment nasal device of the present invention with a non-uniform wall thickness polymeric material; and
Figure 13 is a sectional view of an alternate preferred embodiment with an outer material layer.

### DETAILED DESCRIPTION OF THE PREFERRED EMBODIMENTS

The following description of the preferred embodiment(s) is merely exemplary in nature and is in no way intended to limit the invention, its application, or uses.

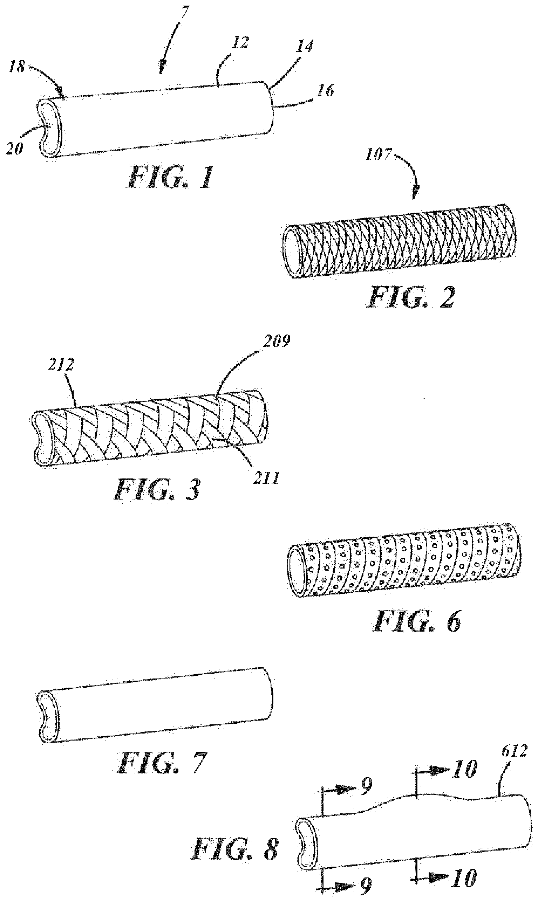

Referring to Figures 1-8, a device 7 to increase airflow in a nasal passage is provided. The device is provided by a hollow tubular member 12 having a posterior end 14 and orifice 16 and anterior end 18 and orifice 20. The anterior end 18 has a slightly larger diameter than the posterior end 14. The tubular member has a wall thickness equal or less than 2 mm and preferably 0.1 to 1.5 mm. The tubular member 12 has a diameter less than that of the nasal passage 10. The tubular member can have a circular, kidney, or other cross-sectional shape.

The tubular member 12 has a shape, which is rigid enough to be self-supporting. Typically the shape of the tubular member is that of a flexible preform. The length of the tubular member 12 will typically be between 25-40 mm depending upon the size of the patient. The tubular member can be selected from a selection of different sized devices or a scan of the patent's nasal passage can be made to customize its size and shape. The tubular member 12 may be a semi-rigid preform or be straight and then be formed by the patients nasal passage.

The tubular member can be expanded by heat. The heat may come from an external heat source, or from heat supplied by the patient's body. In one embodiment, the tubular body 12 can be expanded by irradiation. A particular source of irradiation can be a near-infrared lamp. In still another embodiment the tubular body can be expanded by liquid or gaseous expansion agent. The expansion agent may be inserted into the nasal passage after insertion of the nasal device. The simplest agent being moisture supplied by the patient's body.

In still another embodiment the expansion is mainly provided by a mechanical unwinding and/or expansion of fibers or wires 10 (Figure 2) utilized to reinforce the polymeric material of the tubular member 7 after placement of the nasal device 107 into the patient's nasal passage 10.

In some embodiments, the tubular member 212 (Figure 3) material can be polymeric material fabricated in crisscrossed strands 209, 211 to help to develop a preform shape when expanded.

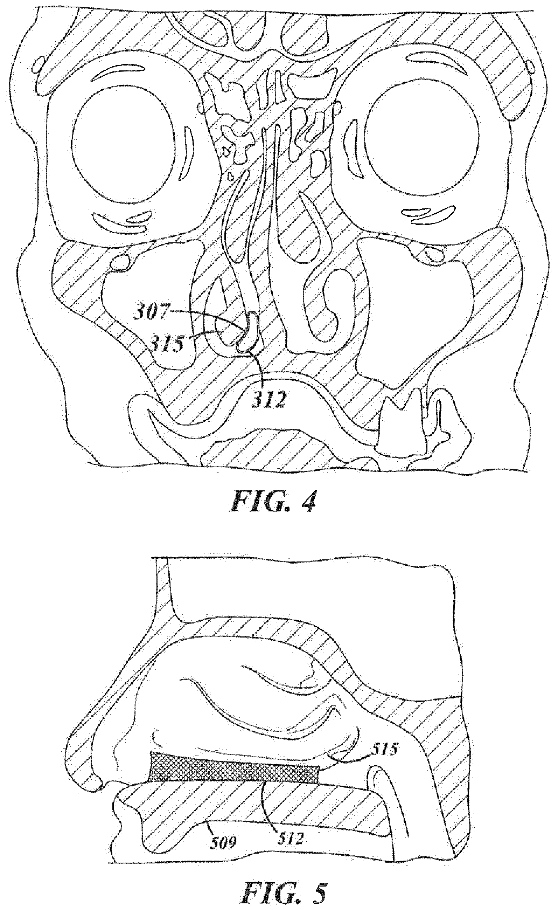

In some embodiments, the tubular member 312 (Figure 4) may be positioned by the nasal epithelium, cartilage and bone 315. In still other applications (Figure 5), tubular member 512 may be placed above hard and soft palate 509 and below the inferior nasal concha 515.

The polymeric material can be a coating on a wire-like enforcement. The wire can be a carbon filament material, polymeric material or metallic material. The reinforcement material may have a fishnet (Figure 2) or spiral design (Figure 6). The reinforcement material can be a thin tubular material (Figure 7). The polymeric material can have openings or perforations, or provide a continuous surface over the reinforcement material. In some applications the reinforcement material or the polymeric material may be bio-absorbable.

In a method of use of the present invention, a scan of the particular nasal cavity is accomplished using known techniques readily available to those skilled in the art. The scan is preferably a full scale size of the nasal cavity in which the tubular member is to be inserted. Using various slices of the cavity the exact shape of the cavity can be dimensioned. With these measurements in hand the tubular member 612 can be custom manufactured to closely match the shape of the cavity in which it will be inserted (Figure 8). This provides expansion of the device to provide a close match to the surfaces in the nasal cavity in order to provide expansion of the device consistent with the contours of the cavity or correct imperfection. This provides a precision matching of the nasal cavity surfaces with the tubular member to avoid any undue stress on the tissues in the nasal cavity.

In yet another method of use of the present invention, the tubular member may have a crescent shape (Figure 4) and be customized for the left or right nostril. The tubular member may be a preform selected from a collection of varying sizes, materials and or flexibilities. The nasal device is placed into the patient's nostril by the patient themselves, by a surgeon or other medically trained personnel. After insertion, the tubular member is then expanded by one or more of the aforementioned methods. The nasal device may be removed by the patient themselves or by medical personnel. The nasal device in many non-surgical applications may be cleansed and inspected to determine reusability. If the nasal device is bio-absorbable, the device is simply allowed to be absorbed into the patient's body.

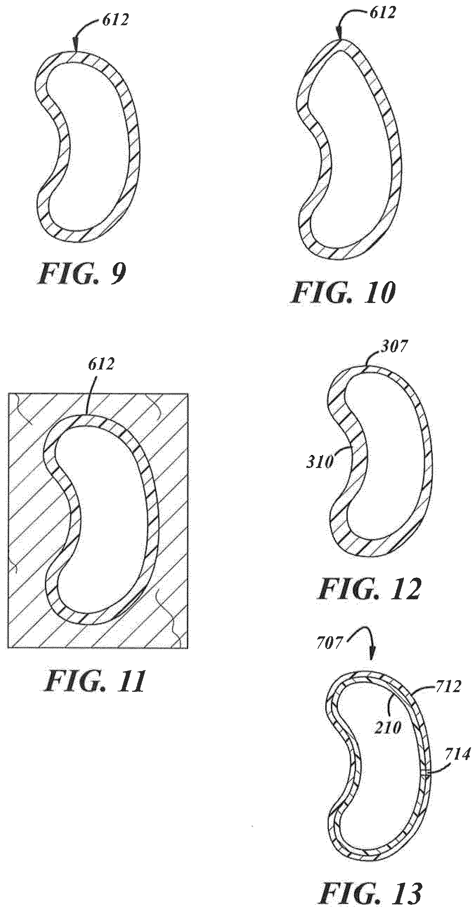

Figure 9 is a sectional view of the customized nasal device of Figure 8 shown prior to expansion. Figure 10 is a sectional view of another portion of the nasal device shown in Figure 10. Figure 11 is a sectional view similar to that of Figure 9 illustrating the nasal device after expansion within a patient's nasal passage 10. Figure 11 is slightly enhanced for purposes of illustration. In most cases the nasal device will be geometrically similar after expansion but it can be fabricated, especially as shown in Figure 3, to be non-geometrically similar when expanded if desired.

Figure 12 is a section view wherein the wall thickness of the polymeric layer is variable. This can be fabricated as a result of scanning of the patient's nostril to add added strength to the nasal insert 307, or the wall 310 can be made thicker to determine expansion shape after enlargement in the patient's nostril. In other embodiments the wall 310 may be larger on pre-sized nasal inserts.

Figure 13 provides an embodiment with an inner polymeric layer 210 and an outer layer 714. The outer layer may be a bio absorbable material or may be impregnated with a pharmaceutical material to prevent infection or to promote healing. One or both of the layers 710, 712 may be penetrated by laser drilled micro or nano sized holes 714 to promote the passage of air or medications. In some embodiments the holes 714 will allow the inner layer to be impregnated with the pharmaceutical material. It is also apparent those skilled in the art that the several features explained in Figures 1-13 can also be combined in a custom embodiment as desired.

In yet still another embodiment of the current invention, the reinforcement material or the polymeric material may have piezoelectric properties to generate expansion within the patient's nasal cavity.

The description of the invention is merely exemplary in nature and, thus, variations that do not depart from the gist of the invention are intended to be within the scope of the invention. Such variations are not to be regarded as a departure from the scope of the invention.

## Claims

1. A device to increase airflow in a nasal passage of a body of a human patient comprising a tubular member (12) being open at extreme cylindrical ends (14, 18), said tubular member (12) having a diameter smaller than a nasal passage accepting said tubular member (12), said tubular member (12) being fabricated at least in part by an expandable polymeric material having a wall thickness equal to or less than 2mm and a length equal to or less than 40mm, **characterized in that** said tubular member (12) is configured to expand intra-nasally by heat, moisture or irradiation.

2. The device of claim 1 wherein said heat is provided by heat from a patient's body, said moisture is provided by a patient's body, and said irradiation is provided by a near-infrared lamp.

3. A device as described in claim 1 wherein said polymeric material is reinforced.

4. A device as described in claim 3 wherein said device is reinforced by a metallic material.

5. A device as described in claim 3 wherein said polymeric material is a coating on a material providing reinforcement and a surface of tubular material has openings.

6. The device of claim 3 wherein said reinforcement material has a wire-like shape and said polymeric material provides a continuous surface over said reinforcement material.

7. A device as described in claim 1 for insertion onto the floor of the nasal cavity.

8. A device as described in claim 1 wherein said tubular member (12) is crescent shaped.

9. A device as described in claim 1 wherein an anterior orifice (20) of said tubular member (12) is larger than a posterior orifice (16) of said tubular member (12).

10. A device as described in claim 1 wherein said tubular member (12) is fabricated from polymeric material in strands, and said strands are crisscrossed so as to expand to a preformed shape.

11. A device as described in claim 3 wherein at least one of said polymeric materials and reinforcement materials is biodegradable.

12. A device as described in claim 3 wherein at least one of said polymeric materials and reinforcement materials is a piezoelectric material.

## Patentansprüche

1. Vorrichtung zum Erhöhen der Luftströmung in einem Nasengang eines Körpers eines menschlichen Patienten, aufweisend ein rohrförmiges Element (12), das an den äußersten zylindrischen Enden (14, 18) offen ist, wobei das rohrförmige Element (12) einen Durchmesser besitzt, der kleiner ist als ein Nasendurchgang, in den das rohrförmige Element (12) eingebracht wird, und wobei das rohrförmige Element (12) zumindest teilweise aus einem ausdehnbaren Polymermaterial hergestellt ist, das eine Wandstärke von kleiner oder gleich 2 mm und eine Länge von kleiner oder gleich 40 mm besitzt, **dadurch gekennzeichnet, dass** das rohrförmige Element (12) dazu ausgebildet ist, dass es sich intranasal durch Wärme, Feuchtigkeit oder Strahlung ausdehnt.

2. Vorrichtung nach Anspruch 1, wobei die Wärme bereitgestellt wird von der Wärme eines Patientenkörpers, und wobei die Feuchtigkeit bereitgestellt wird durch einen Patientenkörper, und wobei die Strahlung bereitgestellt wird durch eine Nah-Infrarotlampe.

3. Vorrichtung nach Anspruch 1, wobei das Polymermaterial verstärkt ist.

4. Vorrichtung nach Anspruch 3, wobei die Vorrichtung durch ein metallisches Material verstärkt ist.

5. Vorrichtung nach Anspruch 3, wobei das Polymermaterial eine Beschichtung auf einem Material ist, das die Verstärkung bereitstellt, und wobei eine Oberfläche des rohrförmigen Materials Öffnungen besitzt.

6. Vorrichtung nach Anspruch 3, wobei das Verstärkungsmaterial eine drahtähnliche Form besitzt und das Polymermaterial eine kontinuierliche Oberfläche auf dem Verstärkungsmaterial vorsieht.

7. Vorrichtung nach Anspruch 1 zum Einsetzen auf den Boden des nasalen Hohlraums.

8. Vorrichtung nach Anspruch 1, wobei das rohrförmige Element (12) sichelförmig ist.

9. Vorrichtung nach Anspruch 1, wobei ein anteriorer Eingang (20) des rohrförmigen Elements (12) größer ist als ein posteriorer Eingang (16) des rohrförmigen Elements (12).

10. Vorrichtung nach Anspruch 1, wobei das rohrförmige Element (12) aus Strängen eines Polymermaterials hergestellt ist, und wobei die Stränge kreuz und quer verlaufen, sodass sie sich zu einer vorgebildeten Form ausdehnen.

11. Vorrichtung nach Anspruch 3, wobei die Polymermaterialien und/oder die Verstärkungsmaterialien biologisch abbaubar sind.

12. Vorrichtung nach Anspruch 3, wobei die Polymermaterialien und/oder die Verstärkungsmaterialien piezoelektrisch sind.

## Revendications

1. Dispositif permettant d'augmenter l'écoulement d'air dans le passage nasal d'un corps d'un patient humain comprenant un élément tubulaire (12) étant ouvert à des extrémités cylindriques extrêmes (14, 18), ledit élément tubulaire (12) ayant un diamètre inférieur à un passage nasal acceptant ledit élément tubulaire (12), ledit élément tubulaire (12) étant fabriqué au moins en partie à partir d'un matériau polymère extensible ayant une épaisseur de paroi inférieure ou égale à 2 mm et une longueur inférieure ou égale à 40 mm, **caractérisé en ce que** ledit élément tubulaire (12) est conçu pour s'étendre de manière intra-nasale par de la chaleur, de l'humidité ou un rayonnement.

2. Dispositif selon la revendication 1, dans lequel ladite chaleur est apportée par de la chaleur provenant du corps d'un patient, ladite humidité est apportée par le corps d'un patient, et ledit rayonnement est apporté par une lampe à infrarouge proche.

3. Dispositif selon la revendication 1, dans lequel ledit matériau polymère est renforcé.

4. Dispositif selon la revendication 3, dans lequel ledit dispositif est renforcé au moyen d'un matériau métallique.

5. Dispositif selon la revendication 3, dans lequel ledit matériau polymère est un revêtement sur un matériau fournissant un renfort et une surface du matériau tubulaire présente des ouvertures.

6. Dispositif selon la revendication 3, dans lequel ledit matériau de renfort a une forme filaire et ledit matériau polymère fournit une surface continue sur ledit matériau de renfort.

7. Dispositif selon la revendication 1 destiné à une insertion sur le sol de la cavité nasale.

8. Dispositif selon la revendication 1, dans lequel ledit élément tubulaire (12) est en forme de croissant.

9. Dispositif selon la revendication 1, dans lequel un orifice antérieur (20) dudit élément tubulaire (12) est supérieur à un orifice postérieur (16) dudit élément tubulaire (12).

10. Dispositif selon la revendication 1, dans lequel ledit élément tubulaire (12) est fabriqué à partir d'un matériau polymère en brins, et lesdits brins sont entrecroisés de manière à s'étendre en une forme préformée.

11. Dispositif selon la revendication 3, dans lequel au moins l'un desdits matériaux polymères et desdits matériaux de renfort est biodégradable.

12. Dispositif selon la revendication 3, dans lequel au moins l'un desdits matériaux polymères et desdits matériaux de renfort est un matériau piézoélectrique.
